# EUROPÄISCHE PATENTANMELDUNG

(11) **EP 1 630 154 A1**
(43) Veröffentlichungstag der Anmeldung: **01.03.2006**
(21) Anmeldenummer: 05017988.6
(22) Anmeldetag: 18.08.2005
(51) Int. Cl.: C07C 209/48, C07C 211/03, C07C 211/21

(54) **Verfahren zur Herstellung von hellfarbenen, primären Fettaminen**

(30) Priorität: 20.08.2004 DE 102004040647
(71) Anmelder: DHW Deutsche Hydrierwerke GmbH Rodleben, 06862 Rodleben (DE)
(72) Erfinder: Haack, Vera, 06844 Dessau (DE); Schröter, Jörg Andreas, 06862 Rosslau (DE); Weidemann, Frank, 06862 Meinsdorf (DE); Steinmeyer, Detlef, 06862 Rosslau (DE)
(74) Vertreter: Tragsdorf, Bodo

(57) **Zusammenfassung**

Die Erfindung bezieht sich auf ein Verfahren zur Herstellung von hellfarbenen, primären Fettaminen, die durch Hydrierung von gesättigten und/oder ungesättigten Fettsäurenitrilen auf der Basis von tierischen und/oder pflanzlichen Fettsäuren mit einer Kettenlänge von 8 bis 22 C-Atomen und dastillativer Aufarbeitung des erhaltenen Hydrierproduktes hergestellt werden, sowie deren Folgeprodukte,

Ausgehend von den Nachteilen des bekannten Standes der Technik ist es Aufgabe, ein wirtschaftliches, einfach auszuführendes Verfahren zur Herstellung von primären, gesättigten und/oder ungesättigten Fettaminen mit hoher Farbstabilität bereitzustellen. Ferner sollen die Fettamlne zur Herstellung hellfarbener Folgeprodukte geeignet sein.

Als Lösung wird vorgeschlagen, dass dem Hydrierprodukt vor der destillativen Aufarbeitung als Additiv Phosphon- und/oder Phosphinsäure und/oder deren Salze, einzeln oder als Gemisch, zugesetzt wird, vorzugsweise in einer Menge von 0,01 bis 5 %, bezogen auf die Menge an Hydrierprodukt, wobei das Additiv in fester Form oder als wässrige Lösung eingesetzt wird.

Durch den Zusatz des Additivs werden als Destillate Fettaminqualitäten erhalten, die deutlich geringere APHA-Farbwerte aufweisen als die entsprechenden unbehandelten Fettamine. Auch unter thermischer Belastung oder während einer längeren zwischenzeitlichen Lagerung des behandelten Fettamins kommt es nur zu einer geringfügigen Erhöhung der APHA-Farbzahl.

## Beschreibung

Die Erfindung bezieht sich auf ein Verfahren zur Herstellung von hellfarbenen, primären, gesättigten und/oder ungesättigten Fettaminen und deren Folgeprodukte.

Es ist bereits seit langem bekannt Fettamine auf Basis tierischer oder pflanzlicher Rohstoffe, ausgehend von den gesättigten oder ungesättigten Fettsäuren oder deren Gemische mit einer Kettenlänge von 8 bis 22 C-Atomen, in die entsprechenden Fettsäurenitrile zu überführen und diese anschließend in flüssiger Phase unter Verwendung geeigneter Katalysatorsysteme zu den Aminen zu hydrieren. Das nach dem Hydrierprozess anfallende Rohamin wird zur Abtrennung von Nebenprodukten abschließend bei Drücken von 1 bis 10 mbar destillativ aufgearbeitet.

Hinsichtlich der allgemein bekannten Verfahrensweise zur Herstellung von Fettaminen auf natürlicher Basis wird auf die DE 40 39 935 A1 und EP 0 490 383 B1 verwiesen. Aliphatische Fettamine auf Basis tierischer oder pflanzlicher Rohstoffe sind u.a. Ausgangsprodukte zur Herstellung von Aminderivaten, wie z.B. Alkoxylaten. Die entsprechenden Fettaminalkoxylate werden in verschiedenen Industriellen Anwendungen als Hilfs- oder Zusatzstoffe eingesetzt, wie z.B. als Antistatika in Polymeren, wobei es von wesentlicher Bedeutung ist, dass die Hilfs- oder Zusatzstoffe helle Farben aufweisen und diese auch über einen langen Zeitraum erhalten bleiben.

Prozess- und rohstoffbedingt sind jedoch die Fettaminalkoxylate meist braune bis hellgelbe Schmelzen, die zum Teil auch Trübungen aufweisen.

Bekannt ist, die gewünschte Farbstabilität durch verfahrenstechnische Maßnahmen und/oder Zusätze auf der Stufe zur Bildung der Fettaminderivate zu erreichen.

Aus der US-PS 3,428,683 ist ein Verfahren zur Alkoxylierung von primären und sekundären Fettaminen bekannt, bei dem in Gegenwart eines niedermolekularen primären oder sekundären Dialkylamins die Umsetzung der Fettamine mit Ethylenoxid erfolgt. Mit diesem Stabilisator erreicht man gelb-braune Ethoxylate.

In der EP 0 477 693 B1 ist ein Verfahren zur Reinigung und Farbstabilisierung von tertiären kurzkettigen N-Alkyldialkanolaminen beschrieben, wobei dem Dialkanolamin eine bestimmte Menge an wasserlöslichem Metallborhydrid zugesetzt wird und eine Vakuumdestillation in Gegenwart von Wasser bei einem Druck von unter 6,67 kPa durchgeführt wird.

Aus der EP 0 690 042 B1 ist ein Verfahren zur Herstellung eines aliphatischen, tertiären Amins bekannt, bei dem das Rohprodukt einem speziellen Reinigungsschritt unterzogen wird. Dieser beinhaltet das Kontaktieren des aliphatischen, tertiären Amins mit einer wässrigen Lösung aus einem anorganischen Alkali, Trennen und Wiedergewinnen des Amins aus der Lösung und/oder Kontaktieren des Amins mit Wasser und abschließendem Abtrennen des Amins vom Wasser. Diese Verfahrensweise kann noch mit einem weiteren Schritt kombiniert werden, nämlich durch Kontaktieren der Produktmischung mittels eines Adsorbens, wie z.B. Aktivkohle, und Trennen und Wiedergewinnung des Amins vom Adsorbens.

Weiterhin ist aus der EP 0 919 537 B1 bekannt, Fettaminalkoxylate durch Umsetzung eines Alkylenoxides mit primären oder sekundären aliphatischen Aminen mit 8 bis 22 C-Atomen herzustellen, wobei vor der Zugabe des Alkylenoxides eine basische Verbindung, wie z.B. NaOH, K₂CO₃, oder eine wässrige Lösung dieser Verbindung zugesetzt wird, die Mischung erhitzt und anschließend das behandelte Amin durch Filtrieren und/oder Destillation abgetrennt wird. Die hier beschriebenen Alkoxylate sollen sich durch eine gute Farbe auszeichnen und lagerstabil sein.

Nachteilig bei den vorgenannten Verfahren ist, dass diese aufwendig (mehrstufig) sind und die zugesetzten Additive sich zum Teil nur schwer abtrennen lassen oder im Produkt verbleiben.

Der vorliegenden Erfindung lag die Aufgabe zugrunde, ein wirtschaftliches, einfach auszuführendes Verfahren zur Herstellung von primären, gesättigten und/oder ungesättigten Fettaminen mit hoher Farbstabilität bereitzustellen.

Ferner sollen die Fettamine zur Herstellung hellfarbener Folgeprodukte geeignet sein. Erfindungsgemäß wird die Aufgabe durch die im Anspruch 1 angegebenen Merkmale gelöst. Vorteilhafte Ausgestaltungen und Weiterbildungen sind Gegenstand der Ansprüche 2 bis 7. Anspruch 8 bezieht sich auf die Verwendung der erfindungsgemäß hergestellten Fettamine zur Herstellung von hellfarbenen Fettaminalkoxylaten.

Gemäß der Aufgabenstellung wurde ein Verfahren zur Herstellung von primären, gesättigten und/oder ungesättigten, heilfarbenen Fettaminen und deren Folgeprodukte gefunden, welches dadurch charakterisiert ist, dass man dem, aus der Hydrierung von gesättigten und/oder ungesättigten Fettsäurenitrilen auf der Basis von tierischen und/oder pflanzlichen Fettsäuren mit einer Kettenlänge von 8 bis 22 C-Atomen resultierendem, katalysatorireiem Rohprodukt vor der destillativen Aufarbeitung als Additiv Phosphon- und/oder Phosphinsäure und/oder deren Salze, einzeln oder als Gemisch, zusetzt und im Anschluss die destillative Aufarbeitung des Hydrierproduktes erfolgt.

Durch den vorgeschlagenen Zusatz von Phosphonsäure und/oder Phosphinsäure und/oder deren Salze, entweder einzeln oder als Gemisch, zu den primären Rohfettaminen, vor deren destillativer Aufarbeitung, werden als Destillate Fettaminqualitäten erhalten, die deutlich geringere APHA-Farbwerte aufweisen als die entsprechenden unbehandelten Fettamine. Auch unter thermischer Belastung oder während einer längeren zwischenzeitlichen Lagerung des behandelten Fettamins kommt es nur zu einer geringfügigen Erhöhung der APHA-Farbzahl. Selbst bei extremen thermischen Belastungen des behandelten Fettamins, wie sie beispielsweise während der Ethoxylierung auftreten, ergeben sich visuell geringfügige Farbvertiefungen.

Primäre Fettamine auf der Basis von tierischen und/oder pflanzlichen Fettsäuren mit einer Kettenlänge von 8 bis 22 C-Atomen, wie z.B. Cocosamin, Talgamin, Stearylamin und Oleylamin, stellen wichtige Ausgangsprodukte zur Herstellung von Fettaminethoxylaten dar, die z.B. als Antistatika für Polymere Verwendung finden. Um zu hellfarbenen Fettaminalkoxylaten zu gelangen, ist es bisher erforderlich, In der Alkoxylierung spezieile Zusatzstoffe einzusetzen, um die gewünschte Produktfarbe zu erreichen.

Durch den Einsatz der erfindungsgemäß erhaltenen, hellfarbenen Fettamine zur Herstellung der Fettaminalkoxylate, kann deren Herstellungsaufwand wesentlich gesenkt werden.

Die Menge des dem Rohamin zugesetzten Additivs beträgt 0,01 bis 5 %, vorzugsweise 0,3 bis 1 %, bezogen auf die Einsatzmenge des aufzuarbeitenden Hydrierproduktes. Aufgrund des in der Praxis besseren Handlings und der einfacheren Dosierung wird das Additiv vorzugsweise in Form einer wässrigen Lösung eingesetzt. Möglich ist auch ein Einsatz als Feststoff.

Als Salze der Phosphon- und/oder Phosphinsäure werden insbesondere Alkali- und Erdalkaliphosphonate bzw. Alkali- und Erdalkaliphosphinate sowie Ammoniumphosphonate und/oder Ammoniumphosphinate als Feststoff oder als wässrige Lösung eingesetzt.

Das Additiv wird vor der destillativen Aufarbeitung des Hydrierproduktes bzw. Rohamins zugesetzt. Es hat sich als vorteilhaft erwiesen, wenn das Hydrierprodukt und dass Additiv vor der destillativen Aufarbeitung bei einer Temperatur von 60 bis 160 °C während einer Zeitdauer von 0,1 bis 10 Stunden vermischt werden, vorzugsweise bei 120 °C während einer Zeitdauer von 2 bis 4 Stunden.

Das Verfahren kann batchweise oder als kontinuierlicher Prozess gestaltet werden. Eventuell nach der Behandlung auftretende Niederschläge und nicht vollständig umgesetzte Phosphorverbindungen können bei Bedarf vor der Vakuumdestillation durch eine einfache Filtration aus dem behandelten Rohprodukt entfernt werden oder verbleiben im Destillationsrückstand.

### Beispiele 1 bis 4

In einem 1-l Rundkolben werden unter Rühren und unter Stickstoff 500 g eines primären Fettamins vorgelegt, das aus der Nitril-Hydrierung stammt und frei an Hydrierkatalysator und Hilfsstoffen ist. Dem jeweiligen Fettamin wird vor der destillativen Aufarbeitung des Rohamins als Additiv Phosphonsäure, 100%ig oder als 50%ige wässrige Lösung, oder Natriumphosphinat zugegeben. Die eingesetzten Fettamine und die Einsatzmengen der Additive sind in der nachfolgenden Tabelle 1 angegeben.

Die Zugabe des Additivs erfolgt unter Rühren, wobei das Gemisch auf eine Temperatur von 120 °C erhitzt und diese Temperatur für ca. zwei Stunden beibehalten wird. Danach wird das Fettamin durch eine einfache Destillation bei einem Druck von 2 mbar und bis zu einer Sumpftemperatur von 180 °C gereinigt. An den erhaltenen Destillaten (Fettaminen) gemäß den Beispielen 1 bis 4 wird die APHA-Farbzahl bestimmt. Für die farbstabilisierten Fettamine sind die ermittelten Farbzahlen in der Tabelle 1 angegeben.

Zur weiteren Prüfung der Farbstabilität der Fettamine unter einer erhöhten thermischen Belastung wird zusätzlich noch ein sogenannter Thermotest durchgeführt, bei dem die einzelnen Fettamine unter einer Stickstoff-Atomsphäre zwei Stunden auf eine Temperatur von 200 °C erhitzt werden. Nach Abschluss des Thermotestes wird an den einzelnen

Proben die APHA-Farbzahl gemäß der vorgenannten Methode bestimmt. Die ermittelten Ergebnisse sind in der Tabelle 1 angegeben.

**Tabelle 1: Mit Additiv behandelte Fettamine**

| Beispiel/Fettamin | Iodzahl | Additiv | | APHA-Farbzahl Destillat | |
|---|---|---|---|---|---|
| | | | Menge (g) | Fettamin | nach Thermotest |
| 1/Cocosamin | 6,7 | Phosphonsäure | 1,5 | 12 | 21 |
| 2/Talgamin, hydriert | 1,5 | 50%ige wässrige Phosphonsäure | 2,5 | 5 | 18 |
| 3/Talgamin | 41,6 | NaH₂PO₂ | 4 | 21 | 27 |
| 4/Oleylamin | 87,5 | 50%ige wässrige Phosphonsäure | 3,0 | 16 | 31 |

### Vergleichsbeispleie V1 bis V4

Analog den erfindungsgemäßen Beispielen 1 bis 4 werden in einem 1-l Rundkolben unter Rühren und unter Stickstoff 500 g eines primären Fettamines vorgelegt, das aus der Nitril-Hydrierung stammt und frei an Hydrierkatalysator und Hilfsstoffen ist.

Anschließend wird das Fettamin durch eine einfache Destillation bei einem Druck von 2 mbar und bis zu einer Sumpftemperatur von 180 °C gereinigt.

Wie bei den erfindungsgemäßen Beispielen 1 bis 4 werden die APHA-Farbzahl vom Destillat sowie nach dem durchgeführten Thermotest ermittelt.

Die Ergebnisse zu den unbehandelten Fettaminen sind in der nachfolgenden Tabelle 2 angegeben.

**Tabelle 2: Unbehandelte Fettamine**

| Beispiel/Fettamin | lodzahl | APHA-Farbzahl Destillat | |
|---|---|---|---|
| | | Fettamin | nach Thermotest |
| V1/Cocosamin | 6,7 | 55 | 98 |
| V2/Talgamin, hydriert | 1,5 | 57 | 75 |
| V3/Talgamin | 41,6 | 32 | 96 |
| V4/Oleylamin | 87,5 | 82 | 108 |

Wie die vorliegenden Ergebnisse zeigen, werden durch den erfindungsgemäßen Zusatz der in Tabelle 1 angegebenen Additive hellfarbene, klare Fettamine mit einer ausgezeichneten Farbstabilität erhalten.

Nachfolgend wird noch an weiteren Beispielen aufgezeigt, dass der Einsatz der erfindungsgemäß hergestellten Fettamine gemäß den Beispielen 1 bis 4 zur Herstellung von Fettaminethoxylaten zu Produkten mit hervorragenden anwendungstechnischen Eigenschaften führt.

### Beispiele 5 bis 8: Herstellung von Fettaminethoxylaten

In einem 2l-Edelstahl-Autoklaven werden 760 g der nach den Beispielen 1 bis 4 hergestellten Fettamine unter Stickstoff vorgelegt und unter Rühren auf eine Temperatur von ca. 165 °C erhitzt. Anschließend wird Innerhalb von zwei Stunden exakt die Menge an Ethylenoxid zudosiert, die notwendig ist, um zwei Mol Ethylenoxid an die Amino-Gruppe anzulagern. Zur vollständigen Umsetzung des Ethylenoxides verbleibt der Reaktionsansatz nach Beendigung der Ethylenoxid-Zufuhr noch weitere 30 Minuten Im Autoklaven. Die ermittelten Farbwerte zu den erhaltenen Fettaminethoxylaten sind in der nachfolgenden Tabelle 3 angegeben. Das Aussehen der Fettaminethoxylate wurde visuell beurteilt und die Farbzahlen bestimmt.

**Tabelle 3: Fettaminethoxylate**

| Beispiel/Ethoxylat | EO-Grad | Aussehen des Ethoxylates | Farbe des Ethoxylates APHA |
|---|---|---|---|
| 5/Cocosamin gemäß Beispiel 1 | 2 | leicht gelbe, klare Schmelze | 98 |
| 6/Talgamin, hydriert gemäß Beispiel 2 | 2 | leicht gelbe, klare Schmelze | 75 |
| 7/Talgamin gemäß Beispiel 3 | 2 | leicht gelbes, klares Öl | 122 |
| 8/Oleylamin gemäß Beispiel 4 | 2 | leicht gelbes, klares Ol | 117 |

### Vergleichsbeispiele V5 bis V8

Unter den gleichen Bedingungen wie in den Beispielen 5 bis 8 wurden unter Verwendung der unbehandelten Fettamine gemäß den Vergleichsbespielen V1 bis V4 die entsprechenden Fettaminethoxylate hergestellt und an diesen die Farbzahlen bestimmt und das jeweils hergestellte Fettaminethoxylat in seinem Aussehen visuell beurteilt. Die Ergebnisse sind in der nachfolgenden Tabelle 4 angegeben.

**Tabelle 4: Mit unbehandeltem Fettamin hergestellte Fettaminethoxylate**

| Beispiel/Ethoxylat | EO-Grad | Aussehen des Ethoxylates | Farbe des Ethoxylates Gardner |
|---|---|---|---|
| V5/Cocosamin gemäß Beispiel V1 | 2 | braune Schmelze | 4,8 |
| V6/Talgamin, hydriert gemäß Beispiel V2 | 2 | braune, leicht trübe Schmelze | 4,5 |
| V7/Talgamin gemäß Beispiel V3 | 2 | braunes Öl | 6,2 |
| V6/Oleylamin gemäß Beispiel V4 | 2 | braunes Öl | 5,5 |

In den vorgenannten Beispielen erfolgte die Bestimmung der APHA-Farbzahl nach der DIN ISO 6271 und die Gardner-Farbzahl nach der DGF C-IV 4c (Farbmessgerät Lico 300, Dr. Lange).

## Patentansprüche

1. Verfahren zur Herstellung von hellfarbenen, primären Fettaminen hergestellt durch Hydrierung von gesättigten und/oder ungesättigten Fettsäurenitrilen auf der Basis von tierischen und/oder pflanzlichen Fettsäuren mit einer Kettenlänge von 8 bis 22 C-Atomen und destillativer Aufarbeitung des erhaltenen Hydrierproduktes, **dadurch gekennzeichnet, dass** dem Hydrierprodukt vor der destillativen Aufarbeitung als Additiv Phosphon- und/oder Phosphinsäure und/oder deren Salze, einzeln oder als Gemisch, zugesetzt wird.

2. Verfahren nach Anspruch 1, **dadurch gekennzeichnet, dass** das Additiv in einer Menge von 0,01 bis 5 %, bezogen auf die Menge an Hydrierprodukt, eingesetzt wird.

3. Verfahren nach einem der Ansprüche 1 oder 2, **dadurch gekennzeichnet, dass** das Additiv in fester Form oder als wässrige Lösung eingesetzt wird.

4. Verfahren nach einem der Ansprüche 1 bis 3, **dadurch gekennzeichnet, dass** als Salze der Phosphon- oder Phosphinsäure Alkaliphosphonate, Erdalkaliphosphonate, Alkaliphosphinate, Erdalkaliphosphinate, Ammoniumphosphonate oder Ammoniumphosphinate eingesetzt werden.

5. Verfahren nach einem der Ansprüche 1 bis 4, **dadurch gekennzeichnet, dass** das Hydrierprodukt und das Additiv vor der destillativen Aufarbeitung bei einer Temperatur von 60 bis 160°C während einer Zeitdauer von 0,1 bis 10 Stunden vermischt werden.

6. Verfahren nach Anspruch 5, **dadurch gekennzeichnet, dass** der Mischprozess bei einer Temperatur von 120 °C während einer Zeitdauer von 2 bis 4 Stunden durchgeführt wird.

7. Verfahren nach einem der Ansprüche 1 bis 6, **dadurch gekennzeichnet, dass** die destillative Aufarbeitung des mit dem Additiv versetzten Hydrierproduktes bei einem Druck von 1 bis 100 mbar erfolgt.

8. Verwendung von hellfarbenen, primären Fettaminen auf der Basis von tierischen und/oder pflanzlichen Fettsäuren mit einer Kettenlänge von 8 bis 22 C-Atomen, die vor der destillativen Aufarbeitung des Hydrierproduktes mit Phosphon- und/oder Phosphinsäure und/oder deren Salze behandelt wurden, als Ausgangsmaterial zur Herstellung von hellfarbenen Fettaminalkoxylaten.
